# FASCICULE DE BREVET EUROPEEN

(11) **EP 2 961 600 B1**
(45) Date de publication et mention de la délivrance du brevet: **06.09.2017**
(21) Numéro de dépôt: 14710042.4
(22) Date de dépôt: 27.02.2014
(51) Int. Cl.: A61L 15/18, A61L 15/24, A61L 15/26, B32B 27/28, C08J 5/12, A61L 15/40, B32B 5/02, B32B 27/12, B32B 27/16, C08J 7/06, A61F 13/02

(54) **ARTICLE COMPRENANT UN SUBSTRAT POLYMERIQUE ET UNE COUCHE DE POLYMERE DE SILICONE**
ARTIKEL MIT EINEM POLYMERSUBSTRAT UND EINER SCHICHT AUS SILIKONPOLYMER
ARTICLE COMPRISING A POLYMERIC SUBSTRATE AND A LAYER OF SILICONE POLYMER

(30) Priorité: 28.02.2013 FR 1351777
(43) Date de publication de la demande: 06.01.2016
(73) Titulaire: Urgo Recherche Innovation et Développement, 21300 Chenove (FR)
(72) Inventeur: PERNOT, Jean-Marc, F-21000 Dijon (FR)
(74) Mandataire: Cabinet Plasseraud
(86) Numéro de dépôt international: PCT/FR2014/050430
(87) Numéro de publication internationale: WO 2014/131999

(56) Documents cités:
- EP-A1- 2 053 161
- EP-A1- 2 327 544
- WO-A1-2005/051442
- WO-A1-2005/102403
- US-B1- 6 475 329
- OLGA GIRSHEVITZ ET AL: "Solution-Deposited Amorphous Titanium Dioxide on Silicone Rubber: A Conformal, Crack-Free Antibacterial Coating", CHEMISTRY OF MATERIALS, vol. 20, no. 4, 1 février 2008 (2008-02-01), pages 1390-1396, XP055087664, ISSN: 0897-4756, DOI: 10.1021/cm702209r

## Description

La présente invention a pour objet un article comprenant un substrat polymérique sur lequel est assemblée une couche de polymère de silicone, l'un au moins de ces deux composés ayant été mis en contact avec des particules de dioxyde de titane, d'oxyde de magnésium et/ou d'oxyde de zinc, et de l'eau avant ou après l'assemblage dudit article.

Les dérivés siliconés sont couramment utilisés dans un certain nombre de domaines techniques comme les domaines cosmétique, aéronautique, automobile ou médical, par exemple pour des revêtements de plastiques ou de textiles, pour des airbags, des prothèses, des implants, des pansements, etc. Les gels ou gommes de silicone présentent notamment l'avantage de pouvoir être assemblés à un grand nombre de supports tout en étant inertes pour l'organisme, évitant ainsi tout problème de toxicité lorsqu'ils sont utilisés chez l'être humain.

Lorsque les dérivés siliconés sont utilisés comme éléments d'enduction de supports ou comme couche apposée sur lesdits supports, il est souvent souhaitable de renforcer leur adhérence sur les substrats sur lesquels ils sont déposés, de manière à ce que les produits ainsi traités résistent efficacement aux contraintes physiques, à l'humidité ou aux variations de température.

Il est par exemple connu de traiter les surfaces des supports ou des surfaces siliconées par traitement Corona pour modifier l'énergie de surface dudit support, de manière à améliorer l'adhérence avec le dérivé siliconé lors de son dépôt. Cependant, les niveaux d'adhérence obtenus par le traitement Corona ne sont pas toujours suffisants, et, dans le cas des surfaces siliconées, ce traitement peut même altérer les caractéristiques adhésives du silicone.

Ainsi, de nouvelles technologies ont été développées pour améliorer l'adhérence entre un substrat polymérique et une couche de polymère siliconé, notamment en mettant en oeuvre des primaires d'accroche.

Il a notamment été décrit dans la littérature de modifier les propriétés d'adhérence de surfaces siliconées par introduction de primaires d'accroche dès l'étape de réticulation du silicone, directement au sein du mélange réactionnel contenant les précurseurs constitutifs du silicone. Il a également été envisagé de déposer ces primaires d'accroche sur un substrat polymérique, substrat sur lequel on vient par la suite couler les précurseurs dudit dérivé siliconé. Dans ces deux cas, le produit final offre des niveaux d'accroche légèrement améliorés par rapport à un produit soumis à un traitement Corona.

Plus récemment, le document WO 2005/051442 de la société Dow Corning Corporation a proposé des méthodes d'amélioration de l'adhérence d'un gel de silicone à la surface d'un substrat polymérique plastique. Cette demande enseigne de traiter directement le substrat ou le silicone déjà synthétisé au moyen de primaires d'accroche de type titanate, et, de façon préférée, de traiter un gel de silicone déjà synthétisé et réticulé.

Ainsi, l'emploi de primaires d'accroche, notamment de primaires de type titanate, a été décrit pour traiter le substrat ou le dérivé siliconé, soit au moment de la réticulation, soit une fois le substrat ou le dérivé siliconé respectivement synthétisé ou réticulé.

Cependant, plus récemment, la demande EP 2 053 161 de la société Dow Corning Toray est venue pondérer en partie l'enseignement de la demande WO 2005/051442 pour ce qui concerne l'adhésion d'une couche de silicone sur un substrat textile. Dans cette demande, il est fait état de l'incapacité pour les primaires d'accroche de type titanate, d'assurer une adhésion prolongée entre un produit textile et une couche de silicone, lorsque ces primaires sont incorporés au sein du mélange réactionnel constitutif de la future couche de silicone. Le produit final obtenu par ce procédé présente des niveaux d'adhérence insuffisants, en particulier lorsqu'il est soumis à des variations importantes de température et d'humidité. Ce document recommande l'utilisation de primaires d'accroche à base de zirconium pour assurer l'adhésion d'une couche de silicone sur un substrat textile.

L'utilisation de primaires d'accroche de type titanate pose, par ailleurs, des problèmes aussi bien lors de leur mise en oeuvre que dans le produit final dans lequel ils sont introduits. En effet, afin d'éviter notamment tout contact avec l'eau de l'air qui pourrait donner naissance à une réaction chimique anticipée, certains de ces primaires d'accroche, parmi lesquels le tétrabutanoate de titane est le plus usité, sont dispersés au sein d'un solvant organique. Cependant, bon nombre de problèmes d'intolérance voire de toxicité liés à l'emploi desdits solvants dans la fabrication d'un article tel que celui objet de la présente invention sont connus. Il est également possible d'utiliser d'autres types de primaires d'accroche fonctionnant en phase aqueuse mais dont les résultats en termes d'amélioration d'adhérence ne sont pas satisfaisants, et nécessitant en outre l'incorporation d'additifs (comme par exemple de l'acide acétique) lors de leur mise en oeuvre et/ou l'application d'un traitement thermique, traitement incompatible avec l'utilisation de certains substrats polymériques.

Ainsi, l'utilisation de primaires d'accroche de différents types et notamment de type titanate, engendre bon nombre de problèmes ou de complications liés à leur mise en oeuvre et à leur utilisation. En outre, les niveaux d'adhérence obtenus par ces traitements nécessitent encore d'être améliorés.

La Demanderesse a ainsi cherché à réaliser un nouveau produit offrant une accroche améliorée entre une couche de polymère de silicone et un substrat polymérique, n'utilisant pas de primaires d'accroche de type titanate, et offrant les meilleures garanties possibles en matière de sécurité sanitaire.

Ainsi, la présente invention a pour objet de proposer de nouveaux articles à base d'un substrat polymérique et d'une couche de polymère de silicone, dans lesquels l'adhérence du silicone sur le substrat est particulièrement élevée, lesdits substrats convenant par ailleurs aux applications médicales ou cosmétiques sans générer de problèmes de toxicité liés à l'emploi de certains solvants.

Plus particulièrement, l'invention a pour objet, selon un premier aspect, un article comprenant au moins un substrat polymérique assemblé à au moins une couche de polymère de silicone, caractérisé en ce que l'un au moins du substrat polymérique ou de la couche de polymère de silicone a été mis en contact avec des particules de dioxyde de titane, d'oxyde de magnésium et/ou d'oxyde de zinc, avant assemblage dudit article, et en ce que l'un au moins du substrat polymérique ou de la couche de polymère de silicone a été mis en contact avec de l'eau avant ou après assemblage dudit article.

De façon tout à fait surprenante, un tel article présente des niveaux d'accroche entre le substrat polymérique, qui est préférentiellement un textile, et la couche de polymère de silicone supérieurs aux articles connus. Ces résultats sont d'autant plus inattendus que le document EP 2 053 161 précédemment cité décourageait strictement d'utiliser des composés à base de titane pour améliorer l'adhérence de produits textiles à un dérivé siliconé.

L'invention a également pour objet, selon un deuxième aspect, une méthode de préparation d'un article tel que décrit précédemment, comprenant les étapes suivantes :
i. la fourniture ou la préparation d'un substrat polymérique,
ii. la fourniture ou la préparation d'une couche de polymère de silicone,
iii. la mise en contact de l'un au moins du substrat polymérique ou de la couche de polymère de silicone avec des particules de dioxyde de titane, d'oxyde de magnésium et/ou d'oxyde de zinc,
iv. la mise en contact de l'un au moins du substrat polymérique ou de la couche de polymère de silicone avec de l'eau avant ou après assemblage dudit article,
v. l'assemblage du substrat et de la couche de polymère de silicone de manière à former ledit article.

L'invention a aussi pour objet, selon un troisième aspect, l'utilisation de particules de dioxyde de titane, d'oxyde de magnésium et/ou d'oxyde de zinc, et de préférence des particules de dioxyde de titane, pour améliorer l'adhérence entre un substrat polymérique et une couche de polymère de silicone.

### Article

La présente invention a pour objet un article comprenant au moins un substrat polymérique assemblé à au moins une couche de polymère de silicone, l'un au moins du substrat polymérique ou de la couche de polymère de silicone, ayant été mis en contact avec des particules de dioxyde de titane, d'oxyde de magnésium et/ou d'oxyde de zinc, avant assemblage dudit article, et l'un au moins du substrat polymérique ou de la couche de polymère de silicone ayant été mis en contact avec de l'eau avant ou après assemblage dudit article.

Alternativement, la présente invention a pour objet un article comprenant au moins un substrat polymérique assemblé à au moins une couche de polymère de silicone, l'un au moins d'un précurseur constitutif du substrat polymérique ou d'un précurseur de la couche de polymère de silicone, ayant été mis en contact avec des particules de dioxyde de titane, d'oxyde de magnésium et/ou d'oxyde de zinc, avant assemblage dudit article.

Par précurseur constitutif du substrat polymérique, on entend tout monomère ou polymère apte à polymériser ou apte à être transformé pour obtenir le substrat fini. Le précurseur peut ainsi être un monomère, un « pré-polymère » ou un polymère.

Par précurseur constitutif de la couche de polymère de silicone, on entend tout composé apte à polymériser pour former la couche de polymère de silicone.

Les articles objets de la présente invention peuvent trouver une application dans un grand nombre de domaines techniques différents tels que le bâtiment, l'aéronautique, l'automobile, le domaine médical ou cosmétique, ou le textile.

L'article selon l'invention peut par exemple être un pansement, un orthèse, une prothèse, un cathéter, un implant, un vêtement, un airbag etc.

Selon un mode préféré de réalisation, l'article selon l'invention est mis en oeuvre dans le domaine médical, et est de préférence un pansement.

### Substrat polymérique

L'article selon la présente invention comprend au moins un substrat polymérique.

On entend par substrat polymérique tout matériau macromoléculaire, notamment obtenu par polymérisation ou transformation d'au moins un précurseur (monomère, pré-polymère ou polymère). Il peut s'agir de matériaux synthétiques ou naturels, par exemple des textiles, des matériaux composites, une association de tous ces matériaux ou encore une association de différents polymères au sein du même matériau et l'association des matériaux ainsi obtenus.

Selon un mode particulier de réalisation, le substrat polymérique mis en oeuvre dans le cadre de la présente invention est une polyoléfine, et peut notamment être choisi parmi la liste non limitative des composés comprenant, entre autres, les polyéthylènes basse densité ou haute densité, les polypropylènes, les polybutylènes, les polyméthylpentènes, et les polymères éthylène-acétate de vinyle (EVA). Le substrat polymérique peut également être choisi parmi la liste non limitative des composés comprenant entre autres les polymères vinyliques tels que les acétates de polyvinyle, l'alcool polyvinylique, le polyvinylbutyral ou le polyvinylformal, les dérivés de chlorure de polyvinyle (chlorure de polyvinyle, chlorure de polyvinylène, le copolymère de polyvinylchloride-propylène) ; les polyuréthanes et les urées-polyuréthanes, les polystyrènes et leurs copolymères (copolymère de styrène-butadiène, de styrène-acrylonitrile, de styrène-butadiène-acrylonitrile), et les dérivés de polymères acryliques (polyacrylates, polyméthacrylate de méthyle, copolymère d'éthylène/acrylate de butyle, copolymère d'éthylène/acrylate de méthyle, copolymère d'éthylène/acide méthacrylique), le polyacrylonitrile, les polyesters (notamment PETE, le polyéthylène téréphtalate, le polybutylène téréphtalate, l'acétate de polyvinyle, les dérivés polylactiques-glycoliques), les films cellulosiques (nitrocellulose, l'éthylcellulose, l'acétate de cellulose, l'acétate butyrate de cellulose, le propionate de cellulose), les polyimides, les polyamides (nylon), les matières plastiques phénoliques et époxy, les polycarbonates, les phénoplastes, les polymères fluorés (polytétrafluoroéthylène, le polyfluorure de vinylidène), les polyoxyméthylènes, les oxydes de polyphénylène, les polysulfones (PSU, PESU, PPSU), le sulfure de polyphényle, et les matériaux à base de polysaccharides.

Le substrat polymérique mis en oeuvre dans la présente invention peut se présenter sous quasiment toutes les formes possibles. Il peut par exemple s'agir d'un film plastique continu ou discontinu, d'un matériau tissé, tricoté ou non tissé, d'un réseau fibreux ou d'une mousse.

Selon un mode préféré de réalisation, le substrat polymérique se présente sous la forme d'un textile, de préférence non tissé, à base de polyéthylène. A titre d'exemple de ce type de substrat, on peut notamment citer les non tissés spun bond constitués de fibres de polyéthylène présentant un grammage compris entre 30 et 40 g/m², tel que celui commercialisé par la société Fiberweb sous la dénomination Berotex-PE® 35g/m² ou celui commercialisé par la société Freudenberg sous la dénomination Vilmed® LSO 1040 WEISS.

Dans le cadre de la présente invention, on préfère utiliser un non tissé, se présentant notamment sous la forme d'un voile « spun laid » présentant un grammage compris entre 5 et 80 g/m², et préférentiellement entre 20 et 50 g/m².

### Polymère de silicone

L'article selon la présente invention comprend également au moins une couche de polymère de silicone.

On parle de « couche » de polymère, dans le cadre de la présente demande, lorsque le polymère de silicone est sous la forme d'une strate continue ou discontinue, d'épaisseur faible par rapport à l'étendue de sa surface.

Le polymère de silicone devrait être choisi de manière à présenter une adhérence optimale sur la peau du patient lorsque l'article est un pansement ou une prothèse. Il devrait notamment être doux et confortable, flexible et résistant, repositionnable et ne pas laisser de résidus sur la peau lors de son retrait.

Le polymère de silicone est de préférence adhésif, physiologiquement acceptable pour la peau, et sa structure est au moins partiellement réticulée, de préférence totalement réticulée.

Parmi les polymères de silicone adhésifs convenant à la présente invention, on distingue les polymères de silicone adhésifs respectant la peau (de l'anglais « soft skin adhesives » (SSA)) et les polymères de silicones adhésifs sensibles à la pression (de l'anglais « pressure sensitive adhesives » (PSA)).

Selon un mode de réalisation préféré de l'invention, le polymère de silicone est un polymère de silicone adhésif respectant la peau (SSA). Ce polymère peut être fabriqué à partir de précurseurs de silicone qui réticulent après avoir été mis en contact suivant une réaction d'hydrosilylation ou de condensation. De tels systèmes sont connus de l'art antérieur, par exemple dans les documents EP-A-0 251 810, EP-A-0 300 620 ou US-A-4 921 704. Les mélanges de précurseurs décrits dans ces documents comprennent pour l'essentiel :
- un composant A qui comprend au moins une polydiméthylsiloxane substituée par un groupe vinyle à chacune de ses extrémités, et un catalyseur de platine, et
- un composant B de polydiméthylsiloxane qui comprend au moins deux groupes hydrogénosilanes.

La mise en présence des deux composants provoque une réaction de réticulation des deux polydiméthylsiloxanes fonctionnalisés qui se produit avantageusement à température ambiante et peut être accélérée par la chaleur.

Les précurseurs du polymère de silicone adhésif peuvent être choisis parmi les produits suivants : Silbione RT Gel® 4712 A&B et Silbione RT Gel® 4717 A&B de Bluestar Silicones, Wacker Silgel® 612 de Wacker-Chemie GmbH, Nusil; MED-6340, Nusil® MED-6345, Nusil® MED3-6300, ou Nusil® MED12-6300 de Nusil Technology, et D-7-9800® de Dow Corning.

Selon un autre mode de réalisation, l'invention met en oeuvre un polymère de silicone adhésif sensible à la pression (PSA). Ce dernier est de préférence obtenu à partir d'une résine de silicone et de silicone fluide. De tels copolymères sont décrits par exemple dans "Silicone Pressure Sensitive Adhesive", Sobieski and Tangney, Handbook of Pressure Sensitive Adhesive Technology (D. Satas Ed.), Von Nostrand Reinhold, New York.

Dans ce mode de réalisation, la résine de silicone est présente à un taux compris entre 45 et 75% (par rapport à la masse totale de silicone) et le silicone fluide est présent à un taux compris entre 25 et 55%, avec la somme des pourcentages de résine de silicone et de silicone fluide qui est égal à 100. De préférence, la résine de silicone est présente à un taux compris entre 55 et 65% (par rapport à la masse totale de silicone) et le silicone fluide est présent à un taux compris entre 35 et 45%, avec la somme des pourcentages de résine de silicone et de silicone fluide qui est égal à 100.

De préférence, la résine de silicone selon l'invention est le produit de condensation de groupements SiO₂ et de groupements R₃(SiO)_{1/2} (triorganosilyl) pour lequel chaque groupement R est indépendamment sélectionné parmi les radicaux méthyle, éthyle, propyle ou vinyl et pour lequel le rapport entre les fonctions SiO₂ et les fonctions R₃(SiO)_{1/2} de la résine de silicone va de 0,6 à 0,9. Des groupements triorganosilyl utilisables pour former la résine de silicone peuvent être des unités triméthylsilyl, triéthylsilyl, méthylméthylproprylsilyl, diméthylvinylsilyl et des mélanges de celles-ci. Le groupement triméthylsilyl est le préféré dans le cadre de l'invention.

De préférence, le silicone fluide selon l'invention est un diorganopolysiloxane aux fonctions OH terminales ayant une viscosité comprise entre 100 et 100000 cSt à 25°C pour laquelle les substituants du diorganopolysiloxane sont indépendamment choisis parmi les radicaux méthyle, éthyle, propyle ou vinyl. Les diorganosiloxances sont, de préférence, des polymères linéaires. Des exemples de diorganopolysiloxane peuvent être, de manière non limitative, un polydiméthylsiloxane, un éthylméthylpolysiloxane, un copolymère de diméthylsiloxane et de méthylvinylsiloxane, et des mélanges de tels polymères ou copolymères ayant des extrémités OH. Le diorganopolysiloxane préféré est un polydiméthylsiloxane.

Des exemples de synthèse d'un tel copolymère sont par exemple décrits dans le brevet US5162410 ou dans le brevet CA711756.

Les copolymères utilisés selon l'invention sont commercialisés par Dow Corning sous la référence BIO-PSA®, ces BIO-PSA® pouvant être sous deux formes, standard ou amine compatible, et étant fournis dans différents solvants avec plusieurs ratio résine de silicone/silicone fluide. On peut notamment citer les grades 7-4400, 7-4500, 7-4600. Le BIO-PSA® particulièrement préféré selon l'invention est le grade 7-4400.

Le polymère de silicone peut comprendre des additifs comme des pigments, des inhibiteurs ou des charges, telles que par exemple des charges de remplissage.

Selon un mode de réalisation particulier, la couche de polymère de silicone est discontinue.

Selon un mode plus préféré de réalisation de l'invention, lorsque l'article est un pansement, la couche de polymère de silicone discontinue peut constituer un enrobage d'une armature ajourée.

L'armature peut être constituée de tout matériau ajouré tels un film perforé, un filet thermoplastique, un textile comme par exemple un tissé, un tricot, ou un non tissé, de préférence élastique pour une meilleure tenue du pansement sur la peau. Un film perforé sera par exemple en polyéthylène ou en polypropylène. Un textile tissé sera par exemple en polyéthylène téréphtalate ou en polyamide. Le grammage de l'armature est, de préférence, compris entre 10 et 500 g/m2, par exemple entre 20 et 300 g/m2.

L'armature peut être enduite du polymère de silicone sur une de ses faces, sur ses deux faces, voire sur toute sa surface. La taille des ouvertures de l'armature peut être comprise entre 0,1 et 5 mm, par exemple entre 0,5 et 3 mm. Selon un mode de mise en oeuvre, le polymère de silicone constitue l'enrobage d'un tricot sur toute sa surface.

### Particules de dioxyde de titane, d'oxyde de magnésium et/ou d'oxyde de zinc

Dans le cadre de la présente invention, l'un au moins du substrat polymérique ou de la couche de polymère de silicone, a été mis en contact avec des particules de dioxyde de titane, d'oxyde de magnésium et/ou d'oxyde de zinc, avant assemblage de l'article objet de la demande, et l'un au moins du substrat polymérique ou de la couche de polymère de silicone a été mis en contact avec de l'eau avant ou après assemblage dudit l'article.

De même, la demanderesse a également envisagé qu'en mettant en contact des précurseurs du substrat polymérique ou des précurseurs de la couche de polymère de silicone avec des particules de dioxyde de titane, d'oxyde de magnésium et/ou d'oxyde de zinc, préalablement ou lors de la synthèse de la couche de polymère de silicone et/ou de la synthèse du substrat polymérique, il était également possible d'augmenter significativement l'adhérence entre les deux éléments une fois synthétisés.

Selon un mode préféré de réalisation, les particules mises en oeuvre dans les articles selon l'invention sont des particules de dioxyde de titane.

La mise en contact du substrat polymérique ou de la couche de polymère de silicone avec des particules de dioxyde de titane, d'oxyde de magnésium et/ou d'oxyde de zinc, en présence d'eau se distingue des traitements connus de l'état de la technique mettant en oeuvre des primaires d'accroche de ces composés, notamment par l'absence de réaction chimique telle que, par exemple, une réaction d'hydrolyse dans le cas des primaires de type tétrabutanoate de titane.

Or la demanderesse a observé, contre toute attente, que des traitements au moyen de particules de dioxyde de titane, d'oxyde de magnésium et/ou d'oxyde de zinc, procuraient des résultats d'adhérence bien supérieurs aux traitements à base de primaires d'accroche, et notamment de type titanate, connus dans l'état de la technique.

Selon un mode de réalisation de l'invention, la couche de polymère de silicone peut être mise en contact avec des particules de dioxyde de titane, d'oxyde de magnésium et/ou d'oxyde de zinc, de préférence par saupoudrage, et le substrat polymérique peut être mouillé, ou humidifié par une solution aqueuse avant assemblage du produit final.

Selon une autre variante de ce mode de réalisation particulier de l'invention, il est possible de mouiller le substrat polymérique une fois celui-ci apposé sur la couche de polymère de silicone comprenant, à sa surface, des particules de dioxyde de titane, d'oxyde de magnésium et/ou d'oxyde de zinc.

Selon un mode particulier de réalisation, pour faciliter le mouillage du substrat polymérique lorsque celui-ci est hydrophobe (polyéthylène notamment), il est possible, dans le cadre de la présente invention, de le soumettre, en outre, à un traitement Corona préalablement à la mise en contact avec de l'eau.

Selon un mode particulièrement préféré de réalisation, l'un au moins du substrat polymérique ou de la couche de polymère de silicone a été mis en contact avec une dispersion aqueuse de particules de dioxyde de titane, d'oxyde de magnésium et/ou d'oxyde de zinc.

Selon un mode de réalisation particulier de l'invention, le substrat polymérique et la couche de polymère de silicone sont tous deux mis en contact avec une dispersion aqueuse de particules de dioxyde de titane, d'oxyde de magnésium et/ou d'oxyde de zinc.

La mise en contact du substrat polymérique ou de la couche de polymère de silicone avec la dispersion aqueuse de particules de dioxyde de titane, d'oxyde de magnésium et/ou d'oxyde de zinc, peut être effectuée par tout moyen connu de l'homme du métier, tels que le sprayage, le foulardage, ou un étalement manuel, comme par exemple à l'aide d'un pinceau.

Les particules de dioxyde de titane, d'oxyde de magnésium et/ou d'oxyde de zinc, présentent notamment une taille allant de 1 nm à 50 µm, de préférence de 1 nm à 10 µm, et plus préférentiellement de 1 nm à 300 nm.

Les particules de dioxyde de titane, d'oxyde de magnésium et/ou d'oxyde de zinc, sont notamment présentes dans la dispersion aqueuse en une teneur allant de 0,05 à 50% en poids, par rapport au poids total de la dispersion, de préférence de 0,1 à 20%, et plus préférentiellement de 0,5 à 5%. En tout état de cause, les particules de dioxyde de titane, d'oxyde de magnésium et/ou d'oxyde de zinc, devraient, de préférence, être présentes en une teneur suffisante pour favoriser l'adhésion du substrat ou de la couche de polymère de silicone sur lesquels elles seront déposées, tout en étant dispersées de manière homogènes dans la phase aqueuse.

La phase aqueuse dans laquelle les particules de dioxyde de titane, d'oxyde de magnésium et/ou d'oxyde de zinc, sont dispersées comprend au moins de l'eau.

La phase aqueuse peut, par ailleurs, comprendre tout autre solvant organique, physiologiquement acceptable, miscible à l'eau.

Selon les connaissances de l'homme du métier, les solvants organiques miscibles à l'eau pouvant être utilisés dans les dispersions aqueuses de particules de dioxyde de titane peuvent permettre d'améliorer l'affinité pour l'eau du substrat polymérique et, de préférence, être volatils, par exemple pour accélérer le séchage du substrat polymérique ou du polymère de silicone après mise en contact.

Parmi les solvants organiques miscibles à l'eau pouvant être utilisés dans le cadre de l'invention, on peut citer notamment les monoalcools inférieurs ayant de 1 à 5 atomes de carbone tels que l'éthanol et l'isopropanol.

Le solvant organique miscible à l'eau, lorsqu'il est présent, devrait être introduit en une teneur limitée pour ne pas de poser de problème de toxicité ou d'irritation. Il peut par exemple être introduit dans la phase aqueuse en une teneur allant de 0,1% à 50% en poids, de préférence allant de 0,5% à 10% en poids, par rapport au poids total de la phase aqueuse.

La dispersion aqueuse peut éventuellement comprendre tout additifs, tels que par exemple des tensioactifs ou des gélifiants hydrophiles, permettant d'améliorer la dispersion des particules de dioxyde de titane, d'oxyde de magnésium et/ou d'oxyde de zinc.

### Méthode de préparation de l'article

L'invention a également pour objet une méthode de préparation d'un article tel que décrit précédemment, comprenant les étapes suivantes :
i. la fourniture ou la préparation d'un substrat polymérique,
ii. la fourniture ou la préparation d'une couche de polymère de silicone,
iii. la mise en contact de l'un au moins du substrat polymérique ou de la couche de polymère de silicone avec des particules de dioxyde de titane, d'oxyde de magnésium et/ou d'oxyde de zinc,
iv. la mise en contact de l'un au moins du substrat polymérique ou de la couche de polymère de silicone avec de l'eau avant ou après assemblage dudit article,
v. l'assemblage du substrat et de la couche de polymère de silicone de manière à former ledit article.

Selon un mode de réalisation de l'invention, dans l'étape iii, c'est la couche de polymère de silicone qui est mise en contact avec les particules de dioxyde de titane, d'oxyde de magnésium et/ou d'oxyde de zinc, de préférence par saupoudrage desdites particules sur la couche de polymère de silicone.

Dans ce mode de réalisation de l'invention, l'étape iv se fait par mouillage du substrat polymérique au moyen d'une solution aqueuse avant assemblage de l'article.

Alternativement, l'étape iv se fait par mouillage du substrat polymérique et de la couche de polymère de silicone au moyen d'une solution aqueuse après assemblage de ces composés pour former l'article.

Ainsi, selon cette alternative, l'invention a pour objet une méthode de préparation d'un article tel que décrit précédemment, comprenant les étapes suivantes :
i. la fourniture ou la préparation d'un substrat polymérique,
ii. la fourniture ou la préparation d'une couche polymère de silicone,
iii. la mise en contact de l'un au moins du substrat polymérique ou de la couche de polymère de silicone avec des particules de dioxyde de titane, d'oxyde de magnésium et/ou d'oxyde de zinc,
iv. l'assemblage du substrat et de la couche de polymère de silicone de manière à former ledit article,
v. la mise en contact du substrat polymérique et de la couche de polymère de silicone avec de l'eau.

Selon un mode particulier de réalisation, pour faciliter le mouillage du substrat polymérique lorsque celui-ci est hydrophobe (polyéthylène notamment), il est possible, dans le cadre de la présente invention, de le soumettre en outre à un traitement Corona préalablement à la mise en contact avec de l'eau.

Ainsi l'étape de fourniture ou de préparation d'un substrat polymérique comprend une étape ultérieure de traitement Corona dudit substrat polymérique préalable à la mise en contact avec de l'eau.

Selon un mode préféré de réalisation, l'invention a également pour objet une méthode de préparation d'un article tel que décrit précédemment, comprenant les étapes suivantes :
i. la préparation d'une dispersion aqueuse de particules de dioxyde de titane, d'oxyde de magnésium et/ou d'oxyde de zinc,
ii. la fourniture ou la préparation d'un substrat polymérique,
iii. la fourniture ou la préparation d'une couche de polymère de silicone,
iv. la mise en contact de la dispersion préparée à l'étape i avec l'un au moins du substrat polymérique ou de la couche de polymère de silicone,
v. l'assemblage du substrat et de la couche de polymère de silicone de manière à former ledit article.

Selon ce mode de réalisation, dans l'étape iv, c'est le substrat polymérique qui est de préférence mis en contact avec la dispersion aqueuse de particules de dioxyde de titane, d'oxyde de magnésium et/ou d'oxyde de zinc. L'étape de mise en contact de la dispersion aqueuse de particules de dioxyde de titane, d'oxyde de magnésium et/ou d'oxyde de zinc, avec le substrat polymérique est alors suivie d'une étape iv' de séchage dudit substrat polymérique imprégné avant assemblage avec la couche de polymère de silicone.

Alternativement, dans l'étape iv, c'est la couche de polymère de silicone qui peut être mise en contact avec la dispersion aqueuse de particules de dioxyde de titane, d'oxyde de magnésium et/ou d'oxyde de zinc. L'étape de mise en contact de la dispersion aqueuse de particules de dioxyde de titane, d'oxyde de magnésium et/ou d'oxyde de zinc, avec la couche de polymère de silicone est alors suivie d'une étape v d'assemblage avec le substrat polymérique, puis d'une étape vi de séchage de l'article ainsi formé.

Selon un mode préféré de réalisation, c'est le substrat polymérique qui est mis en contact avec la dispersion aqueuse de particules de dioxyde de titane, d'oxyde de magnésium et/ou d'oxyde de zinc.

Selon un mode très particulier de réalisation, le substrat polymérique peut être préparé ou fourni dans une première étape i, mis en contact avec la dispersion aqueuse de particules de dioxyde de titane, d'oxyde de magnésium et/ou d'oxyde de zinc, séché ultérieurement, puis la couche de polymère de silicone est réticulée in situ directement sur ledit substrat traité. Dans ce mode de réalisation, les précurseurs constitutifs de la couche de polymère de silicone sont ainsi déposés sur le substrat polymérique, puis réticulés directement sur ledit substrat prétraité avec les particules de dioxyde de titane, d'oxyde de magnésium et/ou d'oxyde de zinc.

La présente invention est illustrée plus en détails dans les exemples de réalisations non limitatifs suivants.

### Exemple

On a préparé trois articles selon l'invention par imprégnation d'un non tissé à base de polyéthylène au moyen d'une dispersion de particules de dioxyde de titane dans un premier cas, d'une dispersion d'oxyde de magnésium dans un deuxième cas et d'une dispersion d'oxyde de zinc dans un troisième cas. On a également préparé un article comparatif par imprégnation d'un non tissé à base de polyéthylène au moyen de primaires d'accroche de type titanate et un article comparatif comprenant un non tissé traité Corona :

### Article selon l'invention comprenant un non tissé imprégné au moyen d'une dispersion de particules de dioxyde de titane

On a préparé une dispersion à 1% en poids de particules de dioxyde de titane (TiO2) dans de l'eau de la manière suivante :
Dans un bécher, on a introduit 198 g d'eau distillée, qu'on a maintenue sous agitation mécanique de manière à créer un vortex.

On a ensuite introduit 2 g de poudre TiO2 de référence commerciale « oxyde de titane transparent PW » commercialisée par la société Sensient et on a maintenu sous agitation de manière à éviter la formation d'agrégats ou d'agglomérats. La dispersion présente une viscosité proche de celle de l'eau.

Une fois la dispersion aqueuse de particules de TiO2 obtenue, on y a fait tremper un non tissé à base de polyéthylène commercialisé sous la dénomination Berotex-PE® 35g/m² par la société Fiberweb, et on a laissé tremper 10 min.

On a ensuite placé le non tissé imprégné entre deux papiers anti-adhérents. On a ensuite comprimé le produit en faisant2 allers-retours avec un rouleau presseur de 10 kg dans le but de simuler l'opération d'exprimage du procédé de foulardage et on a mis le produit sous étuve à 70°C pendant 15min

On a découpé 5 échantillons du substrat polymérique de largeur 50 mm et de longueur 150 mm.

On a découpé 5 échantillons d'une armature ajourée enduite par la couche de polymère de silicone (face adhésive du produit référencé sous la dénomination Novesil 703702 et commercialisé par la société Zodiac Aerospace) de largeur 50 mm et de longueur 150 mm.

On a déposé chaque échantillon de polymère de silicone sur un échantillon de non-tissé et on a comprimé le produit en faisant 2 allers-retours avec un rouleau presseur de 4 kg pour assurer l'adhésion du complexe.

### Article selon l'invention comprenant un non tissé traité Corona et imprégné au moyen d'une dispersion de particules de d'oxyde de magnésium:

On a préparé un second article selon l'invention selon le même protocole en mettant en oeuvre d'une dispersion à 2% en poids de particules d'oxyde de magnésium (MgO) dans de l'eau à la place de la dispersion de dioxyde de titane. En parallèle, on a traité un non tissé polyéthylène commercialisé sous la dénomination Berotex-PE® 35g/m² par la société Fiberweb qui par passage sous une source Corona 0,64 kW à 2 électrodes à une vitesse de 10 m/min à une distance inférieure à 2 mm.

Une fois la dispersion aqueuse de particules de MgO obtenue, on y a fait tremper le non tissé à base de polyéthylène, et on l'a laissé tremper 10 min.

### Article selon l'invention traité Corona comprenant un non tissé imprégné au moyen d'une dispersion de particules de d'oxyde de zinc :

On a également préparé un troisième article selon l'invention selon le même protocole en mettant en oeuvre d'une dispersion à 5% en poids de particules d'oxyde de zinc (ZnO) dans de l'eau à la place de la dispersion de dioxyde de titane.

En parallèle, on a traité un non tissé polyéthylène commercialisé sous la dénomination Berotex-PE® 35g/m² par la société Fiberweb qui par passage sous une source Corona 0,64 kW à 2 électrodes à une vitesse de 10 m/min à une distance inférieure à 2 mm.

Une fois la dispersion aqueuse de particules de MgO obtenue, on y a fait tremper le non tissé à base de polyéthylène, et on l'a laissé tremper 10 min.

### Article comparatif comprenant un non tissé imprégné avec du tétrabutanolate de titane (TnBT) :

On a préparé une solution du tétrabutanoate de titane (TnBT) à 5% en poids dans de l'isopropanol :
Dans un bécher, on a introduit 190 g d'isopropanol, qu'on a maintenue sous agitation mécanique.

On a ensuite introduit 10 g de tétrabutanoate de titane de référence commerciale « Tyzor® TnBT» commercialisée par la société Dorf Ketal et on a maintenu sous agitation et ce jusqu'à complète dissolution.

Une fois la solution de tétrabutanoate de titane obtenue, on y a fait tremper un non tissé à base de polyéthylène commercialisé sous la dénomination Berotex-PE® 35g/m² par la société Fiberweb, et on a laissé tremper 10 min.

On a ensuite placé le non tissé imprégné entre deux papiers anti-adhérents. On a ensuite comprimé le produit en faisant 2 allers-retours avec un rouleau presseur de 10 kg dans le but de simuler l'opération d'exprimage du procédé de foulardage et on a mis le produit sous étuve à 70°C pendant 15min

On a découpé 5 échantillons du substrat polymérique de largeur 50 mm et de longueur 150 mm.

On a découpé 5 échantillons d'une armature ajourée enduite par la couche de polymère de silicone (face adhésive du produit référencé sous la dénomination Novesil 703702 et commercialisé par la société Zodiac Aerospace) de largeur 50 mm et de longueur 150 mm.

On a déposé chaque échantillon de polymère de silicone sur un échantillon de non-tissé et on a comprimé le produit en faisant 2 allers-retours avec un rouleau presseur de 4 kg pour assurer l'adhésion du complexe.

### Article comparatif comprenant un non tissé traité Corona :

Un substrat polymérique de type non tissé polyéthylène commercialisé sous la dénomination Berotex-PE® 35g/m² par la société Fiberweb est traité par passage sous une source Corona 0,64 kW à 2 électrodes à une vitesse de 10 m/min à une distance inférieure à 2 mm.

On a découpé 5 échantillons du substrat polymérique de largeur 50 mm et de longueur 150 mm.

On a découpé 5 échantillons d'une armature ajourée enduite par la couche de polymère de silicone (face adhésive du produit référencé sous la dénomination Novesil 703702 et commercialisé par la société Zodiac Aerospace) de largeur 50 mm et de longueur 150 mm.

On a déposé chaque échantillon de polymère de silicone sur un échantillon de non-tissé et on a comprimé le produit en faisant 2 allers-retours avec un rouleau presseur de 4 kg pour assurer l'adhésion du complexe.

### Test d'adhérence

On a ensuite testé l'adhérence des articles selon l'invention et des deux articles comparatifs préparés ci-dessus, mettant en oeuvre la norme NF EN ISO 11339 - Mai 2010, avec une largeur d'éprouvette de 50 mm et une vitesse d'essai fixée à 300mm/min pour l'essai de pelage en T.

On a laissé les échantillons « climatiser » c'est-à-dire s'acclimater aux conditions de températures et d'hygrométrie du test, pendant 10 minutes dans une salle climatisée.

Pour chaque échantillon d'article, on a désolidarisé le substrat polymérique de la couche de polymère de silicone sur quelques cm à l'une des extrémités.

L'extrémité de la couche de polymère de silicone est pincée dans la mâchoire supérieure du dynamomètre de test, celle du substrat polymérique dans la mâchoire inférieure

On sélectionne une vitesse de traction de 300 mm/min.

On enregistre ensuite la force moyenne nécessaire pour séparer le non tissé de la couche de polymère de silicone pour chaque échantillon.

Pour le décomplexage humide, opéré sur l'article selon l'invention comprenant un non tissé imprégné au moyen d'une dispersion de particules de dioxyde de titane, on place les échantillons 24h à l'étuve à 37°C dans une solution de NaCl, CaCl2.

Après ces 24h, on laisse égoutter 10min avant de faire la mesure.

On enregistre ensuite la force moyenne nécessaire pour séparer le non tissé de la couche de polymère de silicone pour chaque échantillon.

Les résultats sont présentés dans les tableaux suivants :

| | Résultats |
|---|---|
| | Décomplexage sec (cN/5cm) |
| Article selon l'invention (particules de TiO2) | 367,7 |
| Article selon l'invention (particules de MgO) | 333 |
| Article selon l'invention (particules de ZnO) | 369,4 |
| Article comparatif (Tétrabutanoate de titane) | 247,2 |
| Article comparatif (Corona) | 139 |

| | Résultats |
|---|---|
| | Décomplexage humide (cN/5cm) |
| Article selon l'invention (particules de TiO2) | 318,5 |
| Article comparatif (Tétrabutanoate de titane) | 184,9 |
| Article comparatif (Corona) | Non pertinent |

Il apparaît clairement que les articles selon l'invention comprenant un gel de silicone déposé sur un non tissé préalablement mis en contact avec une dispersion aqueuse de TiO2, MgO ou ZnO, présentent une adhérence entre le non tissé et le gel de silicone nettement supérieure aux articles comparatifs comprenant un gel de silicone déposé sur un non tissé traité soit avec un primaire d'accroche tétrabutanoate de titane ou traité Corona.

L'article selon l'invention comprenant un gel de silicone déposé sur un non tissé préalablement mis en contact avec une dispersion aqueuse de TiO2 présente en outre d'excellents résultats lors du test de décomplexage humide.

Il est à noter que, compte tenu des mauvais résultats de décomplexage obtenus à sec avec les substrats traités Corona, le test de décomplexage humide n'a pas été considéré comme pertinent.

## Revendications

1. Article comprenant au moins un substrat polymérique assemblé à au moins une couche de polymère de silicone, **caractérisé en ce que** l'un au moins du substrat polymérique ou de la couche de polymère de silicone, a été mis en contact avec des particules de dioxyde de titane, d'oxyde de magnésium et/ou d'oxyde de zinc, avant assemblage dudit article, et **en ce que** l'un au moins du substrat polymérique ou de la couche de polymère de silicone, a été mis en contact avec de l'eau avant ou après assemblage dudit article.

2. Article selon la revendication 1, **caractérisé en ce que** l'un au moins du substrat polymérique ou de la couche de polymère de silicone, a été mis en contact avec des particules de dioxyde de titane, d'oxyde de magnésium et/ou d'oxyde de zinc, et de l'eau avant assemblage dudit article, de préférence avec une dispersion aqueuse de particules de dioxyde de titane, d'oxyde de magnésium et/ou d'oxyde de zinc.

3. Article selon l'une quelconque des revendications précédentes, **caractérisé en ce que** le substrat polymérique se présente sous la forme d'un textile, de préférence non tissé, à base de polyéthylène.

4. Article selon l'une quelconque des revendications précédentes, **caractérisé en ce que** la couche de polymère de silicone est constituée d'un silicone adhésif, physiologiquement acceptable pour la peau, et sa structure est au moins partiellement réticulée, de préférence totalement réticulée.

5. Article selon l'une quelconque des revendications précédentes, **caractérisé en ce que** le polymère de silicone est obtenu à partir de précurseurs de silicone qui réticulent après leur mise en contact suivant une réaction d'hydrosilylation ou de condensation.

6. Article selon l'une quelconque des revendications précédentes, **caractérisé en ce que** la couche de polymère de silicone est discontinue, ladite couche de polymère de silicone discontinue constituant de préférence l'enrobage d'une armature ajourée.

7. Article selon l'une quelconque des revendications précédentes, **caractérisé en ce que** les particules de dioxyde de titane, d'oxyde de magnésium et/ou d'oxyde de zinc, présentent une taille allant de 1 nm à 50 µm, de préférence de 1 nm à 10 µm, et plus préférentiellement de 1 nm à 300 nm.

8. Article selon l'une quelconque des revendications 2 à 7, **caractérisé en ce que** les particules de dioxyde de titane, d'oxyde de magnésium et/ou d'oxyde de zinc, sont présentes dans la dispersion aqueuse en une teneur allant de 0,05 à 50% en poids, par rapport au poids total de la dispersion, de préférence de 0,1 à 20%, et plus préférentiellement de 0,5 à 5%.

9. Article selon l'une quelconque des revendications 2 à 8, **caractérisé en ce que** la phase aqueuse de la dispersion comprend de l'eau et optionnellement tout autre solvant organique, physiologiquement acceptable, miscible à l'eau.

10. Article selon l'une quelconque des revendications précédentes, **caractérisé en ce qu'**il est mis en oeuvre dans le domaine médical, et est de préférence un pansement.

11. Méthode de préparation d'un article selon l'une quelconque des revendications précédentes, comprenant les étapes suivantes :
i. la fourniture ou la préparation d'un substrat polymérique,
ii. la fourniture ou la préparation d'une couche de polymère de silicone,
iii. la mise en contact de l'un au moins du substrat polymérique ou de la couche de polymère de silicone avec des particules de dioxyde de titane, d'oxyde de magnésium et/ou d'oxyde de zinc,
iv. la mise en contact de l'un au moins du substrat polymérique ou de la couche de polymère de silicone avec de l'eau avant ou après assemblage dudit article,
v. l'assemblage du substrat et de la couche de polymère de silicone de manière à former ledit article.

12. Méthode selon la revendication précédente, comprenant les étapes suivantes :
i. la préparation d'une dispersion aqueuse de particules de dioxyde de titane, d'oxyde de magnésium et/ou d'oxyde de zinc,
ii. la fourniture ou la préparation d'un substrat polymérique,
iii. la fourniture ou la préparation d'une couche de polymère de silicone,
iv. la mise en contact de la dispersion préparée à l'étape i avec l'un au moins du substrat polymérique ou de la couche de polymère de silicone,
v. l'assemblage du substrat et de la couche de polymère de silicone de manière à former ledit article.

13. Méthode selon la revendication 11, **caractérisée en ce que** l'étape iv se fait par mouillage du substrat polymérique et de la couche de polymère de silicone au moyen d'une solution aqueuse après assemblage de ces composés pour former l'article.

14. Méthode selon les revendications 11 ou 12, **caractérisée en ce que** le substrat polymérique préparé ou fourni à l'étape ii subit une étape ultérieure de traitement Corona préalablement à la mise en contact avec de l'eau.

15. Utilisation de particules de dioxyde de titane, d'oxyde de magnésium et/ou d'oxyde de zinc, et de préférence des particules de dioxyde de titane, pour améliorer l'adhérence entre un substrat polymérique et une couche de polymère de silicone.

## Patentansprüche

1. Artikel, welcher wenigstens ein an eine Silikon-Polymerschicht angefügtes Polymersubstrat umfasst, **dadurch gekennzeichnet, dass** wenigstens eines von Polymersubstrat oder Silikon-Polymerschicht vor dem Zusammenfügen des Artikels in Berührung mit Titandioxid-, Magnesiumoxid- und/oder Zinkoxidpartikeln gebracht wurde, und dass wenigstens eines von Polymersubstrat oder Silikon-Polymerschicht vor oder nach dem Zusammenfügen des Artikels in Berührung mit Wasser gebracht wurde.

2. Artikel nach Anspruch 1, **dadurch gekennzeichnet, dass** wenigstens eines von Polymersubstrat oder Silikon-Polymerschicht vor dem Zusammenfügen des Artikels in Berührung mit Titandioxid-, Magnesiumoxid- und/oder Zinkoxidpartikeln und Wasser gebracht wurde, bevorzugt mit einer wässrigen Dispersion mit Titandioxid-, Magnesiumoxid- und/oder Zinkoxidpartikeln.

3. Artikel nach einem der vorhergehenden Ansprüche, **dadurch gekennzeichnet, dass** das Polymersubstrat in Form in Form eines, bevorzugt nicht gewebten, Textils auf Polyethylenbasis vorliegt.

4. Artikel nach einem der vorhergehenden Ansprüche, **dadurch gekennzeichnet, dass** die Silikon-Polymerschicht ein Klebesilikon umfasst, welches für die Haut physiologisch verträglich ist, und dessen Struktur wenigstens teilweise, bevorzugt vollständig, eine Netzstruktur ist.

5. Artikel nach einem der vorhergehenden Ansprüche, **dadurch gekennzeichnet, dass** das Silikon-Polymer ausgehend von Silikonvorläufern erhalten wird, welche nach dem in-Kontakt-bringen nach einer Hydrosilylierungs- oder Kondensierungsreaktion vernetzen.

6. Artikel nach einem der vorhergehenden Ansprüche, **dadurch gekennzeichnet, dass** die Silikon-Polymerschicht unterbrochen ist, wobei die unterbrochene Silikon-Polymerschicht bevorzugt die Umhüllung einer durchbrochenen Struktur bildet.

7. Artikel nach einem der vorhergehenden Ansprüche, **dadurch gekennzeichnet, dass** die Titandioxid-, Magnesiumoxid- und/oder Zinkoxidpartikel eine Größe von 1 nm bis 50 µm, bevorzugt von 1 nm bis 10 µm, und noch stärker bevorzugt von 1 nm bis 300 nm aufweisen.

8. Artikel nach einem der Ansprüche 2 bis 7, **dadurch gekennzeichnet, dass** die Titandioxid-, Magnesiumoxid- und/oder Zinkoxidpartikel in der wässrigen Dispersion mit einem Gehalt von 0,05 bis 50 Gewichtsprozent im Verhältnis zum Gesamtgewicht der Dispersion, bevorzugt mit einem Gehalt von 0,1 bis 20% und noch stärker bevorzugt mit einem Gehalt von 0,5 bis 5%, vorliegen.

9. Artikel nach einem der Ansprüche 2 bis 8, **dadurch gekennzeichnet, dass** die wässrige Phase der Dispersion Wasser umfasst und optional jedes andere physiologisch verträgliche organische Lösungsmittel, das mit Wasser mischbar ist.

10. Artikel nach einem der vorhergehenden Ansprüche, **dadurch gekennzeichnet, dass** er im medizinischen Bereich realisiert ist und bevorzugt ein Verband ist.

11. Herstellungsverfahren eines Artikels nach einem der vorhergehenden Ansprüche, welches die folgenden Schritte umfasst:
i. Bereitstellen oder Herstellen eines Polymersubstrats,
ii. Bereitstellen oder Herstellen einer Silikon-Polymerschicht,
iii. In-Kontakt-bringen von wenigstens einem von Polymersubstrat oder Silikon-Polymerschicht mit Titandioxid-, Magnesiumoxid- und/oder Zinkoxidpartikeln,
iv. In-Kontakt-bringen von wenigstens einem von Polymersubstrat oder Silikon-Polymerschicht mit Wasser vor oder nach dem Zusammenfügen des Artikels,
v. Zusammenfügen des Substrats und der Silikon-Polymerschicht zur Bildung des Artikels.

12. Herstellungsverfahren eines Artikels nach einem der vorhergehenden Ansprüche, welches die folgenden Schritte umfasst:
i. Zubereitung einer wässrigen Dispersion mit Titandioxid-, Magnesiumoxid- und/oder Zinkoxidpartikeln,
ii. Bereitstellen oder Zubereiten eines Polymersubstrats,
iii. Bereitstellen oder Herstellen einer Silikon-Polymerschicht,
iv. In-Kontakt-bringen der in Schritt i hergestellten Dispersion mit wenigstens einem von Polymersubstrat oder Silikon-Polymerschicht,
v. Zusammenfügen des Substrats und der Silikon-Polymerschicht zur Bildung des Artikels.

13. Verfahren nach Anspruch 11, **dadurch gekennzeichnet, dass** der Schritt iv durch Benetzen des Polymersubstrats und der Silikon-Polymerschicht mit einer wässrigen Lösung nach Zusammenfügen dieser Bestandteile zur Bildung des Artikels erfolgt.

14. Verfahren nach den Ansprüchen 11 oder 12, **dadurch gekennzeichnet, dass** das in Schritt ii zubereitete oder bereitgestellte Polymersubstrat einen nachfolgenden Schritt der Corona-Behandlung vor dem in-Kontakt-bringen mit Wasser umfasst.

15. Verwendung von Titandioxid-, Magnesiumoxid- und/oder Zinkoxidpartikeln und bevorzugt Titandioxidpartikeln zur Verbesserung des Anhaftens von einem Polymersubstrat und einer Silikon-Polymerschicht.

## Claims

1. An article comprising at least one polymeric substrate assembled on at least one layer of silicone polymer, **characterized in that** at least one of the polymeric substrate or of the layer of silicone polymer has been brought into contact with particles of titanium dioxide, magnesium oxide and/or zinc oxide, before assembly of said article, and **in that** at least one of the polymeric substrate or of the layer of silicone polymer has been brought into contact with water before or after assembly of said article.

2. The article as claimed in claim 1, **characterized in that** at least one of the polymeric substrate or of the layer of silicone polymer has been brought into contact with particles of titanium dioxide, magnesium oxide and/or zinc oxide, and with water before assembly of said article, preferably with an aqueous dispersion of particles of titanium dioxide, magnesium oxide and/or zinc oxide.

3. The article as claimed in any one of the preceding claims, **characterized in that** the polymeric substrate is in the form of a fabric, preferably nonwoven, based on polyethylene.

4. The article as claimed in any one of the preceding claims, **characterized in that** the layer of silicone polymer consists of a silicone adhesive, physiologically acceptable for the skin, and its structure is at least partially crosslinked, preferably fully crosslinked.

5. The article as claimed in any one of the preceding claims, **characterized in that** the silicone polymer is obtained from silicone precursors that crosslink after they are brought into contact, by a hydrosilylation reaction or a condensation reaction.

6. The article as claimed in any one of the preceding claims, **characterized in that** the layer of silicone polymer is discontinuous, said discontinuous layer of silicone polymer preferably constituting the coating of an openwork frame.

7. The article as claimed in any one of the preceding claims, **characterized in that** the particles of titanium dioxide, magnesium oxide and/or zinc oxide have a size ranging from 1 nm to 50 µm, preferably from 1 nm to 10 µm, and more preferably from 1 nm to 300 nm.

8. The article as claimed in any one of claims 2 to 7, **characterized in that** the particles of titanium dioxide, magnesium oxide and/or zinc oxide are present in the aqueous dispersion in a content ranging from 0.05 to 50 wt%, relative to the total weight of the dispersion, preferably from 0.1 to 20%, and more preferably from 0.5 to 5%.

9. The article as claimed in any one of claims 2 to 8, **characterized in that** the aqueous phase of the dispersion comprises water and optionally any other physiologically acceptable water-miscible organic solvent.

10. The article as claimed in any one of the preceding claims, **characterized in that** it is used in the medical field, and is preferably a dressing.

11. A method of preparing an article as claimed in any one of the preceding claims, comprising the following steps:
i. supplying or preparing a polymeric substrate,
ii. supplying or preparing a layer of silicone polymer,
iii. bringing at least one of the polymeric substrate or of the layer of silicone polymer into contact with particles of titanium dioxide, magnesium oxide and/or zinc oxide,
iv. bringing at least one of the polymeric substrate or of the layer of silicone polymer into contact with water before or after assembly of said article,
v. assembling the substrate and the layer of silicone polymer so as to form said article.

12. The method as claimed in the preceding claim, comprising the following steps:
i. preparing an aqueous dispersion of particles of titanium dioxide, magnesium oxide and/or zinc oxide,
ii. supplying or preparing a polymeric substrate,
iii. supplying or preparing a layer of silicone polymer,
iv. bringing the dispersion prepared in step i into contact with at least one of the polymeric substrate or of the layer of silicone polymer,
v. assembling the substrate and the layer of silicone polymer so as to form said article.

13. The method as claimed in claim 11, **characterized in that** step iv is carried out by wetting the polymeric substrate and the layer of silicone polymer by means of an aqueous solution after assembly of these compounds to form the article.

14. The method as claimed in claims 11 or 12, **characterized in that** the polymeric substrate prepared or supplied in step ii undergoes a subsequent step of corona treatment before being brought into contact with water.

15. The use of particles of titanium dioxide, magnesium oxide and/or zinc oxide, and preferably particles of titanium dioxide, for improving the adherence between a polymeric substrate and a layer of silicone polymer.
